# EUROPEAN PATENT APPLICATION

(11) **EP 4 362 034 A1**
(43) Date of publication of application: **01.05.2024**
(21) Application number: 23205898.2
(22) Date of filing: 25.10.2023
(51) Int. Cl.: G16H 10/60, G16H 30/20, G06F 3/0481, G06F 16/176, G06F 16/27, G06Q 90/00

(54) **MEDICAL INFORMATION PROCESSING APPARATUS AND MEDICAL INFORMATION PROCESSING SYSTEM**

(30) Priority: 28.10.2022 JP 2022173033
(71) Applicant: Canon Medical Systems Corporation, Tochigi 324-0036 (JP)
(72) Inventor: MITSUMORI, Keita, Otawara-shi, 324-0036 (JP)
(74) Representative: Marks & Clerk LLP

(57) **Abstract**

A medical information processing apparatus (100) according to an embodiment includes an assignment unit (143) and a setting unit (144). Based on files pertaining to medical information that users intend to browse or operate and folders that store the files therein, which are managed by each user, the assignment unit (143) assigns a label representing an attribute to each of the folders. Based on the files stored in the folders with the same or similar label, the setting unit (144) sets a condition pertaining to presentation of the file.

## Description

### FIELD

Embodiments described herein relate generally to a medical information processing apparatus and a medical information processing system.

### BACKGROUND

In one of the conventionally known techniques, medical information such as information about particular diseases is shared among a plurality of medical facilities with personal information including patients' names anonymized for use in diagnosis and researches, etc. Physicians and others who browse such medical information may use a bookmark function, which creates a folder with a desired name and stores and manages files for the medical information (or links to access such files) in the folder.

### SUMMARY OF THE INVENTION

A medical information processing apparatus includes:
an assignment unit configured to, based on files pertaining to medical information that users intend to browse or operate, and folders that store the files therein, the files and the folders being managed by the respective users, assign a label representing an attribute to each of the folders; and
a setting unit configured to, based on the files stored in the folders with the labels with the same attribute, set a condition pertaining to presentation of the file.

The setting unit may be configured to, based on a first file corresponding to the file that a part of first users among the users stores in the folder, set a condition to present an action of adding the first file to the folder to second users who do not have the first file stored in the folder.

The medical information processing apparatus may further include a presentation unit configured to present an action of a file operation pertaining to the presentation of the file to the second user. The presentation unit may be configured to present the action of adding the first file to the folder to the second user, based on the set condition.

The medical information processing apparatus may further include an acquisition unit configured to acquire operation information representing a content of an operation of the user for the file. The presentation unit is configured to present the action to the second user, based on the operation information of the first user in the operation information acquired about the users and the set condition that is set.

The acquisition unit may be configured to acquire the operation information expressing the content of the file operation for the file or the folder that stores a link capable of accessing the file.

The medical information processing apparatus may further include:
a specification unit configured to specify the attribute representing a characteristic of the folder, based on a name of the folder as a target of the file operation included in the operation information; and
a counting unit configured to count the number of labels, for each attribute, assigned to the files or the folders storing the files, for each file. The assignment unit may be configured to assign the label representing the attribute specified by the specification unit to each of the folders and the files stored in the folders. The counting unit may be configured to count the number of labels with the same attribute assigned to the files. Based on the counted number of labels with the same attribute assigned to the files and the condition that is set, the presentation unit may be configured to present the action of the file operation for the folder to the second user.

The setting unit may be configured to set, as the condition, that an increase quantity of the number of labels with the same attribute assigned to the first file in a predetermined period is over a threshold. When the condition is satisfied, the presentation unit may be configured to present the action of adding the first file to the folder to the second user who manages the file different from the first file using the folder assigned with the label with the same attribute as the label set in the condition.

The setting unit may be configured to set, as the condition, that a decrease quantity of the number of labels with the same attribute assigned to a second file corresponding to the file that a part of the second users stores in the folder, in a predetermined period is over a threshold. When the condition is satisfied, the presentation unit may be configured to present an action of deleting the second file from the folder to the second user who manages the second file using the folder assigned with the label with the same attribute as the label set in the condition.

The medical information processing apparatus may further include an extraction unit configured to extract a keyword representing a characteristic of the file from the file. The specification unit may be configured to specify the attribute, based on the keyword extracted by the extraction unit.

The medical information processing apparatus may further include a retrieval unit configured to retrieve a relevant file related to a third file that the second user stores in the folder. When, as the condition, the relevant file in which the number of labels with the same attribute increases over the threshold in the predetermined period exists, the presentation unit may be configured to present an action of adding the relevant file to the folder to the second user who does not have the relevant file stored in the folder.

The presentation unit may be configured to derive the action of the file operation, based on the operation information of the first user, in the operation information acquired about the users, assuming that the user who performs the file operation for the file more frequently than or as frequently as a threshold is the first user.

A medical information processing system includes a memory apparatus configured to store medical information therein, one or a plurality of terminal apparatuses configured to, by using a folder created for each user and being capable of storing the medical information or a link to the medical information as a file, receive a file operation of the user for the file, and a medical information processing apparatus. The medical information processing apparatus includes:
an assignment unit configured to, based on files pertaining to medical information that users intend to browse or operate, and folders that store the files therein, the files and the folders being managed by the respective users, assign a label representing an attribute to each of the folders; and
a setting unit configured to, based on the files stored in the folders with the labels with the same attribute, set a condition pertaining to presentation of the file.

### BRIEF DESCRIPTION OF THE DRAWINGS

FIG. 1 is a block diagram illustrating one example of a structure of a medical information processing system according to one embodiment;
FIG. 2 is a diagram for describing one example of a bookmark display screen in the embodiment;
FIG. 3 is a diagram for describing one example of the relation among data, labels, and a score in the embodiment;
FIG. 4 is a diagram for describing one example of a label assignment process in the embodiment;
FIG. 5 is a diagram for describing one example of a recommendation process for data addition in the embodiment;
FIG. 6 is a diagram for describing one example of a recommendation process for data deletion in the embodiment;
FIG. 7 is a diagram for describing one example of a recommendation process for adding data with a different label in the embodiment;
FIG. 8 is a diagram for describing one example of a display control process for a terminal apparatus in the embodiment;
FIG. 9 is a diagram for describing one example of a label assignment process when the assignment of the label by the analysis of a name of a bookmark folder is impossible in the embodiment;
FIG. 10 is a diagram for describing one example of the label assignment process when the assignment of the label by the analysis of the name of the bookmark folder is impossible in the embodiment;
FIG. 11 is a diagram for describing one example of the recommendation process for users who do not use the bookmark function in the embodiment;
FIG. 12 is a flowchart expressing one example of a process for a user who uses the bookmark function to be performed by a medical information processing apparatus according to the embodiment; and
FIG. 13 is a flowchart expressing one example of a process for a user who does not use the bookmark function to be performed by the medical information processing apparatus according to the embodiment.

### DETAILED DESCRIPTION

Embodiments of the medical information processing apparatus and a medical information processing system will be described below in detail with reference to the drawings.

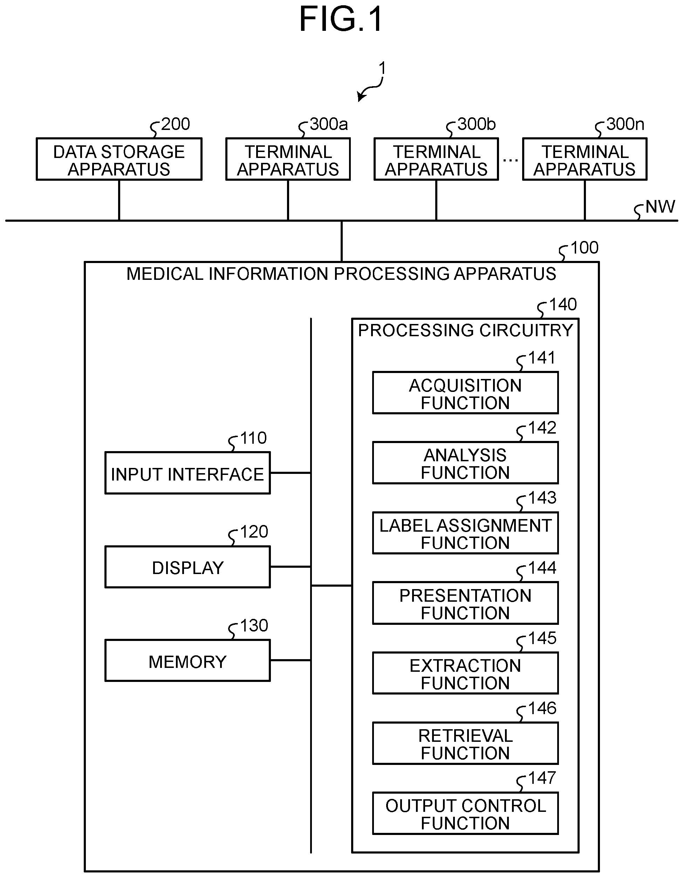

A medical information processing system 1 illustrated in FIG. 1 is described below as one example. FIG. 1 is a block diagram illustrating one example of a structure of the medical information processing system 1 according to the embodiment.

As illustrated in FIG. 1, the medical information processing system 1 includes a medical information processing apparatus 100, a data storage apparatus 200, and a plurality of terminal apparatuses 300 (300a, 300b, ... 300n). The medical information processing apparatus 100, the data storage apparatus 200, and the terminal apparatuses 300 are communicatively connected via a network NW.

The medical information processing apparatus 100, the data storage apparatus 200, and the terminal apparatuses 300 may be installed in the same facility or in different facilities. That is, the network NW may be formed by a local network closed within the hospital, or may be a network via the Internet.

The medical information processing apparatus 100, for example, manages files pertaining to medical information specified by a bookmark function to be described below (hereinafter also referred to as "files" simply) or folders that store such files therein by assigning labels to the files and folders. Here, the label refers to information that indicates the attribute of the file or the folder that stores the file therein.

The medical information processing apparatus 100 is an apparatus that induces an action that is recommended with respect to the file, which is triggered by an activity based on the user's operation on the folder, the file, or the label. The medical information processing apparatus 100 is realized by a computer appliance such as a workstation. The medical information processing apparatus is discussed below.

The data storage apparatus 200 is a memory apparatus that stores files therein. The data storage apparatus 200 is realized by a computer appliance such as a database (DB) server, and stores files in a semiconductor memory element such as a RAM or a flash memory, or in a memory such as a hard disk or an optical disk. In this embodiment, the data storage apparatus 200 stores files across multiple medical facilities therein.

Here, examples of the files include medical information such as electronic medical record data, examination result data, medical image data generated by various kinds of modalities, data on side effects of treatment, and data from papers on cases. The files are stored in the data storage apparatus 200 with the patient information, such as patient names, contained in the file anonymized.

Although only one data storage apparatus 200 is illustrated in FIG. 1, the medical information processing system 1 may include more than one data storage apparatus 200. For example, the medical information processing system 1 may include the data storage apparatus 200 as an electronic medical record storage apparatus, the data storage apparatus 200 as a specimen examination server, the data storage apparatus 200 as a PACS server, etc.

The terminal apparatus 300 is an information processing apparatus to be used by the user. Here, the user may be a physician or a co-medical staff member such as a nurse, a pharmacist, a clinical laboratory technician, a radiology technician, a physical therapist, a speech therapist, or a dental hygienist. The terminal apparatus 300 is realized by a computer appliance such as a desktop personal computer (PC), a laptop PC, or a tablet PC. Information such as a user ID identifying the user and a terminal ID identifying the terminal apparatus 300 is stored in the memory apparatus of the terminal apparatus 300.

For example, the terminal apparatus 300 communicates with the data storage apparatus 200 via the network NW and causes, for example, a display apparatus of the terminal apparatus 300 to display the files stored in the data storage apparatus 200. This allows the user to browse the files stored in the data storage apparatus 200.

By the way, the user will browse various files (hereinafter, files are also referred to as data) for diagnostic and research purposes. In addition, the same data may be browsed many times, and it is very time-consuming to retrieve the data each time. In view of this, the user may use a bookmark function. The bookmark function is a managing function to enable the user who wants to browse the medical information that the user browsed before, to easily access that medical information.

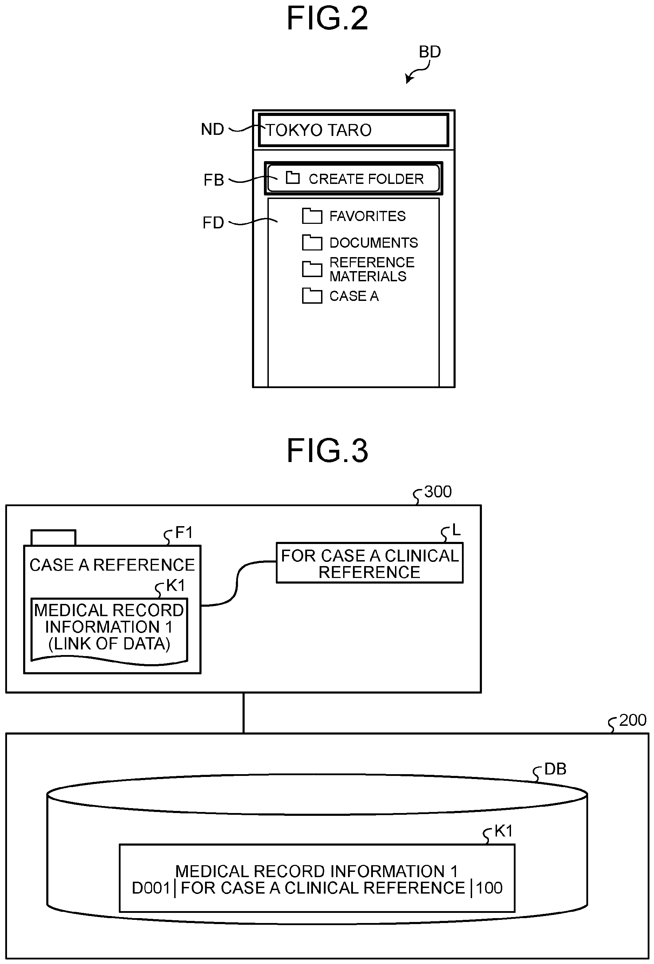

Here, FIG. 2 is a diagram for describing one example of a bookmark display screen. A bookmark display screen BD includes a user name display column ND, a folder creation button FB, and a folder display column FD. The user name display column ND is a display column where the name of the user is displayed. Although only the user's name "Tokyo Taro" is displayed in FIG. 2, the user ID identifying the user, the information indicating the user's attribute (for example, clinician, researcher, etc.), and the like may be displayed in addition to the user's name.

The folder creation button FB is a button for creating a folder for storing and managing files. The user, for example, creates folders with desired names that suit the intended use of the data via the terminal apparatus 300 and manages the data. Hereinafter, the folder created in the bookmark display screen BD is also referred to as a bookmark folder.

The folder display column FD is a display column where the bookmark folder is displayed. In FIG. 2, four folders are displayed: "favorites," "documents," "reference materials," and "case A".

The user stores data in the respective bookmark folders and manages the data. In this embodiment, a copy of the data stored in the data storage apparatus 200 is stored in the bookmark folder; however, a link to access the data may alternatively be stored in the bookmark folder. Additionally, subfolders may be stored within the bookmark folder.

Referring back to FIG. 1, the medical information processing apparatus 100 is described. The medical information processing apparatus 100 includes an input interface 110, a display 120, a memory 130, and a processing circuitry 140, as illustrated in FIG. 1.

The input interface 110 receives various input operations from an operator (for example, an administrator of the medical information processing apparatus 100), converts the received input operations into electrical signals, and outputs the electric signals to the processing circuitry 140.

For example, the input interface 110 can be realized by a mouse, a keyboard, a trackball, switches, buttons, a joystick, a touchpad where an input operation is performed by a touch on an operation surface, a touchscreen that integrates a display screen and a touchpad, a non-contact input circuitry using an optical sensor, a voice input circuitry, or the like.

The input interface 110 may be configured by a tablet terminal or the like capable of wireless communication with the medical information processing apparatus 100. The input interface 110 is not limited only to those with physical operation components such as a mouse and a keyboard. For example, a processing circuitry for electrical signals that receives electrical signals corresponding to input operations from an external input appliance provided separately from the medical information processing apparatus 100 and outputs these electrical signals to the processing circuitry 140 is also an example of the input interface 110.

The display 120 displays various kinds of information. In one example, the display 120 displays examination orders and examination results. In another example, the display 120 displays a graphical user interface (GUI) for receiving various operations from the user. The display 120 is, for example, a liquid crystal display or a cathode ray tube (CRT) display.

The display 120 may be a desktop type or may be configured by a tablet terminal or the like capable of wireless communication with the medical information processing apparatus 100. The input interface 110 and the display 120 may be integrated. For example, the input interface 110 and the display 120 are realized by a single touch panel.

The medical information processing apparatus 100 may include a plurality of displays 120. For example, the medical information processing apparatus 100 may include two physically separated displays (dual display) as the display 120. These multiple displays 120 may also be controlled to be related with each other.

For example, the displays 120 are controlled to display one continuous region. In this case, the display region in the display 120 is extended according to the number of displays 120.

For example, the memory 130 is realized by a semiconductor memory element such as a random access memory (RAM) or a flash memory, a hard disk, an optical disc, or the like. In one example, the memory 130 stores therein computer programs for each circuitry in the medical information processing apparatus 100 to realize its functions. In another example, the memory 130 stores therein label setting information to be described below.

The processing circuitry 140 controls the entire processing by the medical information processing apparatus 100 by executing an acquisition function 141, an analysis function 142, a label assignment function 143, a presentation function 144, an extraction function 145, a retrieval function 146, and an output control function 147.

Note that the acquisition function 141 is one example of an acquisition unit. The presentation function 144 is one example of a setting unit, a counting unit, and a presentation unit. Note that the analysis function 142 is one example of a specification unit. The label assignment function 143 is one example of an assignment unit. The extraction function 145 is one example of an extraction unit. The retrieval function 146 is one example of a retrieval unit.

The acquisition function 141 acquires operation information indicating the contents of the user's file operations. Specifically, the acquisition function 141 monitors the user's operations performed on the bookmark folder by communicating with the terminal apparatus 300 via the network NW.

For example, when the data stored in the medical information processing apparatus 100 is stored in the bookmark folder, the acquisition function 141 acquires information about the data to be stored and information about the bookmark folder in which the data is stored, as the operation information indicating the contents of the user's file operation.

Specifically, the acquisition function 141 acquires, from the terminal apparatus 300, information such as the user ID of the user who stores the data, the terminal ID of the terminal apparatus 300, the date and time when the data is stored (the data is operated), the information expressing the name of the stored data, the information expressing the name of the bookmark folder in which the data is stored, and the record that the data is stored in the bookmark folder as the operation information.

In another example, in a case where the data stored in the bookmark folder is deleted, the acquisition function 141 acquires, from the terminal apparatus 300, the information such as the user ID of the user who deletes the data, the terminal ID of the terminal apparatus 300, the date and time when the data is deleted (the data is operated), the information expressing the name of the deleted data, the information expressing the name of the bookmark folder in which the deleted data used to be stored, and the record that the data is deleted from the bookmark folder as the operation information.

The analysis function 142 analyzes the name of the bookmark folder. For example, the analysis function 142 analyzes the name of the bookmark folder included in the operation information acquired by the acquisition function 141. A known natural language processing technique or the like can be used for the analysis. Based on the name of the bookmark folder as the target of the file operation included in the operation information, the analysis function 142 specifies the attribute that represents the characteristic of the bookmark folder and the data stored in the bookmark folder.

For example, the analysis function 142 specifies the attribute of the bookmark folder and data pertaining to the operation information acquired by the acquisition function 141, based on the label setting information stored in the memory 130 and the analysis results.

Here, the label setting information is, for example, information that defines the name of the label representing the attribute of the bookmark folder or data. With reference to the label setting information stored in the memory 130, the analysis function 142 specifies the name of the label corresponding to the analysis result of the name of the bookmark folder as the attribute of the bookmark folder as the target of the operation and the data stored in that bookmark folder.

Based on the file that the user intends to browse or operate and the bookmark folder that stores the file therein, which are managed by each of the users, the label assignment function 143 assigns the label representing the attribute to the bookmark folder.

For example, the label assignment function 143 assigns the label representing the attribute specified by the analysis function 142 to the bookmark folder pertaining to the operation information acquired by the acquisition function 141. For example, the label assignment function 143 assigns the label representing the attribute specified by the analysis function 142 to the data pertaining to the operation information acquired by the acquisition function 141. The label assignment function 143 may assign multiple labels to a single piece of data.

Based on the file stored in the bookmark folder of a non-target user, who is not the target to which the file is presented, the presentation function 144 sets a threshold of the number of assigned labels (hereinafter also referred to as score) to be presented to a target user to which the file is presented and who does not have that file stored in his or her bookmark folder. The non-target user is one example of first users. The target user is one example of second users.

Here, the threshold of the score is one example of conditions pertaining to the presentation of a file. For example, the presentation function 144 determines the threshold of the score according to the total number of users having the bookmark folder to which a particular label is assigned.

The presentation function 144 counts the score for each label assigned, for each file. Based on the counted score and the set score threshold, the presentation function 144 presents to the target user, an action of adding the file that the non-target user has stored in his or her bookmark folder to the bookmark folder of the target user.

The presentation function 144 presents an action of the file operation to the target user, based on the operation information of the non-target user in the operation information acquired about the users, and the set score threshold.

For example, if there is data whose label score has increased over the set threshold, the presentation function 144 specifies the users who manage the data using the bookmark folder with the same label as the label assigned to that data.

The presentation function 144 then generates a notification to encourage the target user, among the specified users, who does not have the data to store the data in the bookmark folder. The "data whose score has increased over the set threshold" in the above case is one example of a first file.

With reference to FIG. 3, the relation among the data, the labels, and the score is described below. FIG. 3 is a diagram for describing one example of the relation among the data, the labels, and the score. As illustrated in FIG. 3, the database DB is stored in the memory apparatus of the data storage apparatus 200 or the like. The database DB stores therein, as the data, electronic medical record data K1 with the file name "medical record information 1" (hereinafter also referred to as "medical record information 1" K1), for example.

The "medical record information 1" K1 has as ancillary information, a data identifier "D001" that identifies the data, a label "for case A clinical reference", and the score "100". The combination of the data identifier and the label makes the data unique.

On the other hand, a bookmark folder F1 with the folder name "for case A reference" (hereinafter also referred to as "case A reference" F1) is created in the terminal apparatus 300 by the user's operation, for example. To "case A reference" F1, a label L with the label name "for case A clinical reference" is assigned by the label assignment function 143.

In addition, "case A reference" F1 contains a link K1l to the electronic medical record data with the file name "medical record information 1". For example, by clicking the link to the data with a mouse or the like, the user can access the data stored in the data storage apparatus 200 corresponding to the link. The bookmark folder may store therein a copy of the data stored in the data storage apparatus 200.

Here, the score of the label represents, for example, the number of identical labels assigned to the data. Thus, data assigned with two or more kinds of labels will have a score for each label assigned.

For example, if there is data whose label score has decreased over a set threshold, the presentation function 144 specifies the users who manage the data using the bookmark folder with the same label as the label assigned to that data.

The presentation function 144 then generates a notification to encourage the target user, among the specified users, who has that data stored in the bookmark folder to delete the data from the bookmark folder. The "data whose score has decreased over the set threshold" in the above case is one example of a second file.

The user who performs the file operations more frequently than or as frequently as a threshold is the non-target user, and based on the operation information of the non-target user in the operation information acquired about the users, the presentation function 144 derives the action of the file operation to be presented to the target user.

For example, the presentation function 144 calculates the frequency of the file operations on the basis of the user ID and the data operation date and time included in the operation information. The presentation function 144 then specifies the user whose calculated frequency is more than or equal to the threshold as the non-target user. The presentation function 144 then derives the action of the file operation to be presented to the target user on the basis of the operation information of the non-target user.

Here, the user who frequently performs the file operations is considered to be a user who manages the data as appropriate. Therefore, by deriving the action to be presented to the target user on the basis of the operation information of the non-target user who performs the file operations more frequently than or as frequently as a threshold, it is possible to induce the action similar to that for the non-target user who is considered to manage the data as appropriate with respect to the target user even if the target user is the user, for example, who tends to neglect the data management.

From the file, the extraction function 145 extracts a keyword that characterizes that file. For example, if the attribute of the bookmark folder cannot be specified from the name of the bookmark folder, the analysis function 142 sends to the extraction function 145, information that the attribute of the bookmark folder could not be specified.

When the information that the attribute of the bookmark folder could not be specified is sent from the analysis function 142, the extraction function 145 extracts the keyword from the data pertaining to the operation information acquired by the acquisition function 141. The known natural language processing technique or the like can be used to extract keywords. The extraction function 145 sends the extraction result as a summary to the analysis function 142.

In this case, the analysis function 142 specifies the attribute of the bookmark folder on the basis of the label setting information stored in the memory 130 and the summary sent out by the extraction function 145. Thus, even when the label to be assigned to the data could not be specified in the analysis of the name of the bookmark folder, the label can be assigned to the bookmark folder and the data stored in the bookmark folder.

The retrieval function 146 retrieves relevant files associated with the files stored in the bookmark folder. For example, if the data is electronic medical record data, the retrieval function 146 retrieves the electronic medical record data of patients who are strongly correlated with the patient pertaining to that electronic medical record data as the relevant data.

In the above case, the electronic medical record data originally stored in the bookmark folder is one example of third file. The relevant data is one example of the relevant file. The strongly correlated patient is, for example, a similar patient who is similar to a particular patient in terms of disease name, attribute, etc. For example, the retrieval function 146 retrieves the data assigned with the same label, as the relevant data.

The output control function 147 outputs information to the terminal apparatus 300. For example, the output control function 147 sends the notification generated by the presentation function 144 to the terminal apparatus 300 via the network NW. Thus, addition of data or deletion of data can be recommended to the target user.

The output control function 147 also sends to the terminal apparatus 300, a control instruction to change the display order of the data in the bookmark folder according to the label score. This allows, for example, the data in the bookmark folder to be sorted in order of label score, so that the user can easily grasp the importance of the data.

In the medical information processing apparatus 100 illustrated in FIG. 1, each processing function is stored in the memory 130 in the form of a computer program executable by a computer. The processing circuitry 140 is a processor that reads out each computer program from the memory 130 and executes the computer program to realize the function corresponding to the computer program. In other words, the processing circuitry 140 that has read out each computer program has a function corresponding to the read computer program.

Although one processing circuitry 140 performs the acquisition function 141, the analysis function 142, the label assignment function 143, the presentation function 144, the extraction function 145, the retrieval function 146, and the output control function 147 in the description based on FIG. 1, the processing circuitry 140 may alternatively be configured by a combination of a plurality of independent processors and each processor may execute the computer program to achieve the function. The processing functions of the processing circuitry 140 may be distributed or integrated into a single or multiple processing circuitry as appropriate.

The term "processor" used in the above description means, for example, a central processing unit (CPU), a graphics processing unit (GPU), an application specific integrated circuit (ASIC), a programmable logic device (for example, simple programmable logic device (SPLD)), a complex programmable logic device (CPLD), a field programmable gate array (FPGA), and other circuitry. The processor reads out and executes a computer program saved in the memory 130 to realize the function.

In FIG. 1, one memory 130 is described as storing computer programs corresponding to the respective processing functions. However, the embodiment is not limited to this configuration. For example, a plurality of the memories 130 may be dispersed, and the processing circuitry 140 may be configured to read the corresponding computer programs from the respective memories 130. Instead of saving the computer program in the memory 130, the computer program may be incorporated directly into the circuitry of the processor. In this case, the processor reads out and executes the computer program incorporated in the circuitry to realize the function.

The processing circuitry 140 may use a processor of an external apparatus connected via a network to achieve the function. For example, the processing circuitry 140 reads out the computer programs corresponding to the respective functions from the memory 130 and executes the computer programs, and additionally uses a server group (cloud) connected to the medical information processing apparatus 100 via the network as a calculation source, thereby achieving the respective functions illustrated in FIG. 1.

The overall structure of the medical information processing system 1 according to the embodiment has been described. Under such a structure, the medical information processing system 1 in a first embodiment assigns labels to the data in the bookmark folder created by the bookmark function and presents the action of the file operation for the data on the basis of the pieces of operation information for the files assigned with the same label. The operation of each function of the medical information processing system 1 in the embodiment is described below.

In the following description, users who use the bookmark function are classified into three categories: meticulous users, somewhat meticulous users, and non-meticulous users.

In this embodiment, the meticulous user means a user who names the bookmark folders in such a way that their use and purpose can be understood, and who frequently organizes the files stored in the bookmark folders.

The somewhat methodical user means a user who names the bookmark folders in such a way that their use and purpose can be understood, but tends to neglect the files stored in the folders.

The non-meticulous user means a user who names the bookmark folders in such a way that their use and purpose cannot be understood, and tends to neglect the files stored in bookmark folders.

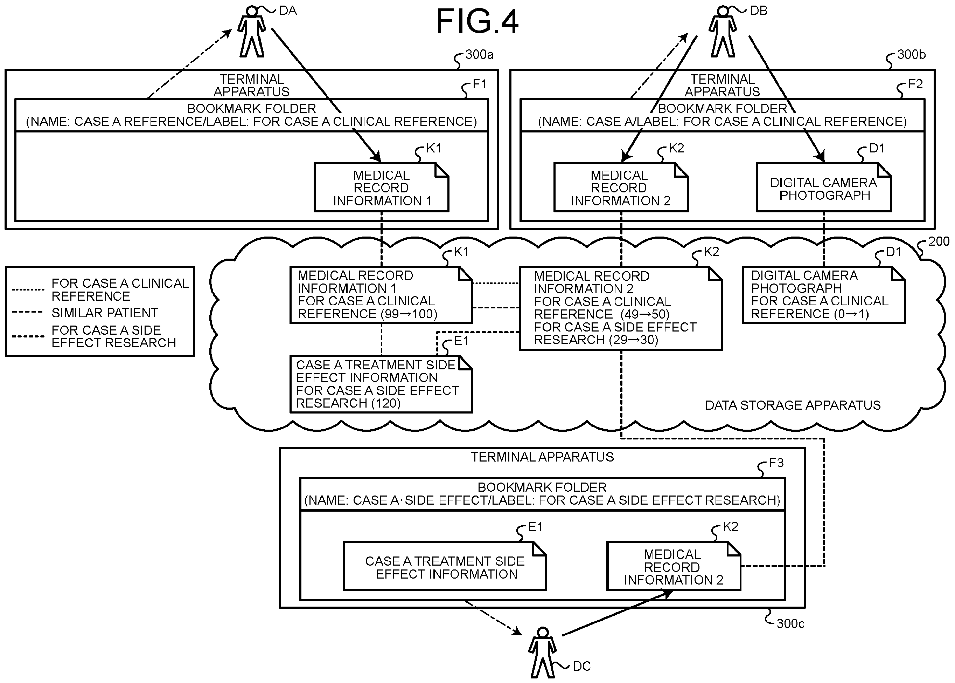

First, a label assignment process for the data is described with reference to FIG. 4. FIG. 4 is a diagram for describing one example of the label assignment process. In FIG. 4, the medical information processing system 1 includes the medical information processing apparatus 100 (not illustrated), the data storage apparatus 200, and the terminal apparatuses 300a, 300b, and 300c. The lines connecting the data in the data storage apparatus 200 and the data in the terminal apparatus 300 in the diagram represent the operation of the medical information processing apparatus 100.

In FIG. 4, "medical record information 1" K1, electronic medical record data K2 with the file name "medical record information 2" (hereinafter also referred to as "medical record information 2" K2), image data D1 with the file name "digital camera photograph" (hereinafter also referred to as "digital camera photograph" D1), and side effect data E1 with the file name "case A side effect information" (hereinafter also referred to as "case A treatment side effect information" E1) are stored as the data in the data storage apparatus 200.

"Medical record information 1" K1, "medical record information 2" K2, "digital camera photograph" D1, and "case A treatment side effect information" E1 are examples of the files.

The label "for case A clinical reference" is assigned to "medical record information 1" K1 in the data storage apparatus 200. The score of the label "for case A clinical reference" in "medical record information 1" K1 is assumed to be "99".

The labels "for case A clinical reference" and "for case A side effect research" are assigned to "medical record information 2" K2 in the data storage apparatus 200. The score of "for case A clinical reference" in "medical record information 2" K2 is assumed to be "49". The score of "for case A side effect research" in "medical record information 2" K2 is assumed to be "29".

In addition, "digital camera photograph" D1 in the data storage apparatus 200 is not assigned with the label and has a label score of 0. Furthermore, the label "for case A side effect research" is assigned to "case A treatment side effect information" E1 in the data storage apparatus 200. The score of "for case A side effect research" of "case A treatment side effect information" E1 is assumed to be "120".

In FIG. 4, the users who use the bookmark function are physicians DA, DB, and DC. The physician DA is a clinician and a somewhat methodical user. The physician DB is a clinician and a meticulous user. The physician DC is a research physician and a methodical user.

The terminal apparatus 300a is a terminal apparatus used by the physician DA. Similarly, the terminal apparatus 300b is a terminal apparatus used by the physician DB. Similarly, the terminal apparatus 300c is a terminal apparatus used by the physician DC.

In the terminal apparatus 300a, "case A reference" F1 is created. A bookmark folder F2 with the folder name "case A" (hereinafter also referred to as "case A" F2) is created in the terminal apparatus 300b.

A bookmark folder F3 with the folder name "case A/side effect" (hereinafter also referred to as "case A/side effect" F3) is created in the terminal apparatus 300c. In addition, "case A treatment side effect information" E1 is stored in "case A treatment/side effect" F3.

Although illustrated in FIG. 4, in the initial state, "case A reference" F1 in the terminal apparatus 300a and "case A" F2 in the terminal apparatus 300b are not assigned with the label (the empty state). The label "for case A side effect research" is assigned to "case A/side effect" F3 in the terminal apparatus 300c.

Each piece of data stored in the data storage apparatus 200 has a data identifier, which is not illustrated in FIG. 4. The data identifier is also omitted in the following drawing.

Based on the above premise will be described an example of operation in a case where the physician DA adds "medical record information 1" K1 stored in the data storage apparatus 200 to "case A reference" F1 via the terminal apparatus 300a.

In this case, the acquisition function 141 of the medical information processing apparatus 100 detects that "medical record information 1" K1 has been added to "case A reference" F1 in response to the operation of the physician DA. The acquisition function 141 then acquires information indicating that "medical record information 1" K1 has been added to "case A reference" F1.

Next, the analysis function 142 performs an analysis of "case A reference" F1, which is the name of the bookmark folder. Furthermore, the analysis function 142 specifies the attribute of "medical record information 1" K1 with reference to the label setting information stored in the memory 130. For example, the analysis function 142 specifies "for case A clinical reference" registered in the label setting information as the attribute of "medical record information 1" K1 by absorbing fluctuations in the notation of "case A reference" F1 using the natural language processing technique or other means.

Then, the label assignment function 143 assigns the label "for case A clinical reference" representing "for case A clinical reference" to "medical record information 1" K1. In addition, the label assignment function 143 updates the score of the label "for case A clinical reference" in "medical record information 1" K1 stored in the data storage apparatus 200 as a result of the assignment of the label. In FIG. 4, the label assignment function 143 adds one to the score of "for case A clinical reference" in "medical record information 1" K1, and updates the score from "99" to "100".

The label assignment function 143 assigns the label "for case A clinical reference" representing the attribute of "case A reference" F1 to "case A reference" F1 because the label has not been assigned to the "case A reference" F1.

Next described is an example of operation in a case where the physician DB adds "medical record information 2" K2 and "digital camera photograph" D1 stored in the data storage apparatus 200 to the bookmark folder "case A" F2 via the terminal apparatus 300b. In this case, each function of the medical information processing apparatus 100 also performs the process similar to that when "medical record information 1" K1 is added to "case A reference" F1 described above.

As a result, the score of "for case A clinical reference" in "medical record information 2" K2 is updated from "49" to "50" and the score of "for case A clinical reference" in "digital camera photograph" D1 is updated from "0" to "1".

If the data contains character strings, such as electronic medical record data, it is possible to analyze the character strings in the data and assign the labels to the bookmark folders, but the labels that do not suit the intended use or purpose may be assigned.

For example, the data stored in "case A reference" F1 or "case A" F2 illustrated in FIG. 4 is considered not to be limited to direct evidence and materials for the case A. In another example, it is also considered that another case accompanying the case A or a case of side effect due to treatment is stored in these bookmark folders. In such cases, if labels are assigned by analyzing the character strings in the data, the suitable label, that is, "for case A clinical reference," may not be assigned.

On the other hand, the medical information processing apparatus 100 in this embodiment regards the name of the bookmark folder as the attribute of the data stored in that bookmark folder, and by analyzing the name of the bookmark folder, can specify the label to be assigned to the data. In other words, the medical information processing apparatus 100 according to this embodiment can assign the suitable label to the data that does not contain character strings or that is not directly related to the name of the bookmark folder.

Next described is an example of operation in a case where the physician DC adds "medical record information 2" K2 stored in the data storage apparatus 200 to "case A/side effect" F3 via the terminal apparatus 300c.

In this case, the acquisition function 141 detects that "medical record information 2" K2 has been added to "case A/side effect" F3 in response to the operation of the physician DC. The acquisition function 141 then acquires information indicating that "medical record information 2" K2 has been added.

Next, the analysis function 142 detects that the label "for case A side effect research" has been assigned to "case A/side effect" F3. The analysis function 142 sends to the label assignment function 143, information that the label "for case A side effect research" has been assigned to "case A/side effect" F3.

Then, the label assignment function 143 assigns to "medical record information 2" K2, the label "for case A side effect research", which is the same label as "case A/side effect" F3 in which "medical record information 2" K2 is stored. In addition, the label assignment function 143 updates the score of "for case A side effect research" in "medical record information 2". In FIG. 4, the label assignment function 143 adds one to the score of "for case A side effect research" in "medical record information 2" and updates this score from "29" to "30".

In FIG. 4, two labels, "for case A clinical reference" and "for case A side effect research", are assigned to "medical record information 2" K2. Thus, more than one label may be assigned to some data. In the following description, such data may be referred to as hub data. The hub data is used for processes, for example, to encourage the user to add the data assigned with the label different from the label assigned to the data pertaining to the user's behavior. The hub data is discussed below.

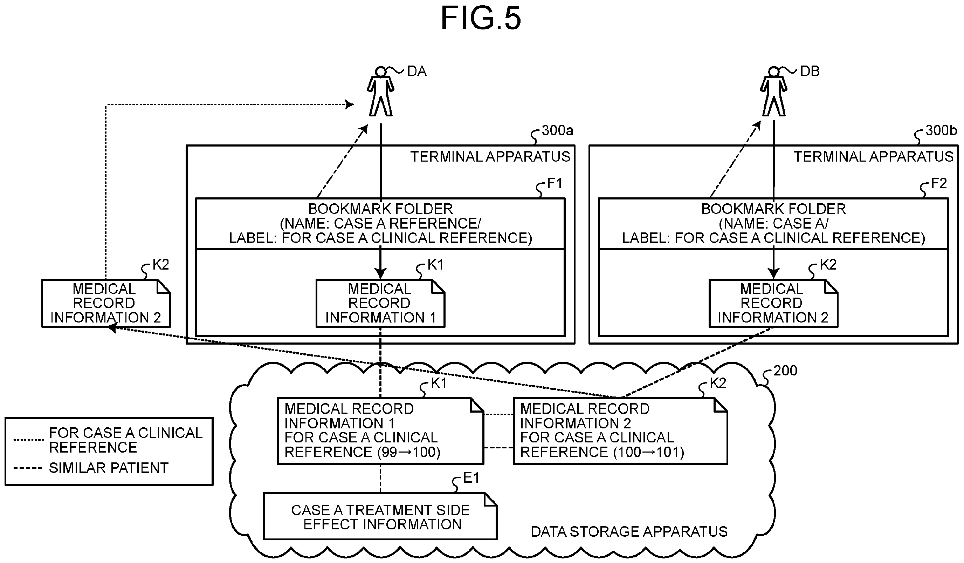

Next, a recommendation process for the data addition is described with reference to FIG. 5. FIG. 5 is a diagram for describing one example of the recommendation process for the data addition.

In FIG. 5, the users who use the bookmark function are the physician DA and the physician DB. In a manner similar to FIG. 4, "medical record information 1" K1 is added to "case A reference" F1 by the physician DA and "medical record information 2" K2 is added to "case A" F2 by the physician DB. Moreover, the label "for case A clinical reference" is assigned to "case A reference" F1 in the terminal apparatus 300a and "case A" F2 in the terminal apparatus 300b.

The score of "for case A clinical reference" in "medical record information 1" K1 is assumed to be "99". The score of "for case A clinical reference" in "medical record information 2" K2 is assumed to be "100".

In this case, the label assignment function 143 updates the score of "for case A clinical reference" in "medical record information 1" K1 from "99" to "100" and updates the score of "for case A clinical reference" in "medical record information 2" K2 from "100" to "101".

The retrieval function 146 retrieves the relevant data of "medical record information 1" K1 pertaining to the operation information of the physician DA. In FIG. 5, the retrieval function 146 retrieves the data of similar patients who are similar to the patient pertaining to "medical record information 1" K1 as the relevant data. The fact that the patient is the similar patient can be determined, for example, from the patient information and chief complaint of the patient pertaining to "medical record information 1" K1. In FIG. 5, the retrieval function 146 retrieves the data assigned with the same label as the label "for case A clinical reference" assigned to "medical record information 1" K1 as the relevant data.

Here, "medical record information 2" K2 is the data of the similar patient who is similar to the patient pertaining to "medical record information 1" K1. In addition, "medical record information 2" K2 is the data assigned with the label "for case A clinical reference" that is the same as the label of "medical record information 1" K1. In addition, "case A treatment side effect information" E1 is the data of the similar patient who is similar to the patient pertaining to "medical record information 1" K1.

Therefore, "medical record information 2" K2 and "case A treatment side effect information" E1 are retrieved as the relevant data of "medical record information 1" K1 by the retrieval function 146. The retrieval function 146 associates "medical record information 1" K1 and "medical record information 2" K2 to the effect that they are the data of the similar patients. Similarly, the retrieval function 146 associates "medical record information 1" K1 and "case A treatment side effect information" E1 to the effect that they are the data of the similar patients.

Furthermore, since "medical record information 1" K1 and "medical record information 2" K2 are assigned with the same label "for case A clinical reference", the retrieval function 146 associates "medical record information 1" K1 and "medical record information 2" K2 to the effect that they are the data assigned with the same label. Although not illustrated, the retrieval function 146 performs the process similar to that for "medical record information 1" K1 pertaining to the operation information of the physician DA as for "medical record information 2" K2 pertaining to the operation information of the physician DB.

The presentation function 144 then presents the operation action for the data. In FIG. 5, the presentation function 144 checks the change in label score in a predetermined period regarding the data that is associated to the effect that the data is assigned with the label similar to that of "medical record information 1" K1.

For example, in FIG. 5, the score of "for case A clinical reference" in "medical record information 2" K2 increases from "100" to "101". The presentation function 144 checks such a change in score in a predetermined period (for example, from one month ago to the present time).

The presentation function 144 determines, for example, whether the increase quantity in the score of "for case A clinical reference" in "medical record information 2" K2 in the predetermined period is over a predetermined threshold. If the increase quantity in the score of "for case A clinical reference" in the predetermined period is over the threshold, the presentation function 144 specifies the users who manage the data using the bookmark folder assigned with the label "for case A clinical reference".

The presentation function 144 also generates a notification to encourage the user, among the specified users, who does not have "medical record information 2" K2 to add "medical record information 2" K2 to that bookmark folder.

In FIG. 5, the physician DA manages the data using "case A reference" F1, which is the bookmark folder assigned with the label "for case A clinical reference". In addition, the physician DA does not have "medical record information 2" K2. Therefore, the presentation function 144 generates a notification to encourage the physician DA to add "medical record information 2" K2 to "case A reference" F1.

If "medical record information 2" K2 is stored in the bookmark folder that is not "case A reference" F1, the presentation function 144 may generate a notification to encourage the user to move "medical record information 2" K2 to "case A reference" F1.

Here, the data with the increasing label score is considered to be the data that is referenced by many users. Thus, as described above, the presentation function 144 encourages the user to store the data with the increasing label score in the bookmark folder, thereby preventing the user from overlooking highly important information.

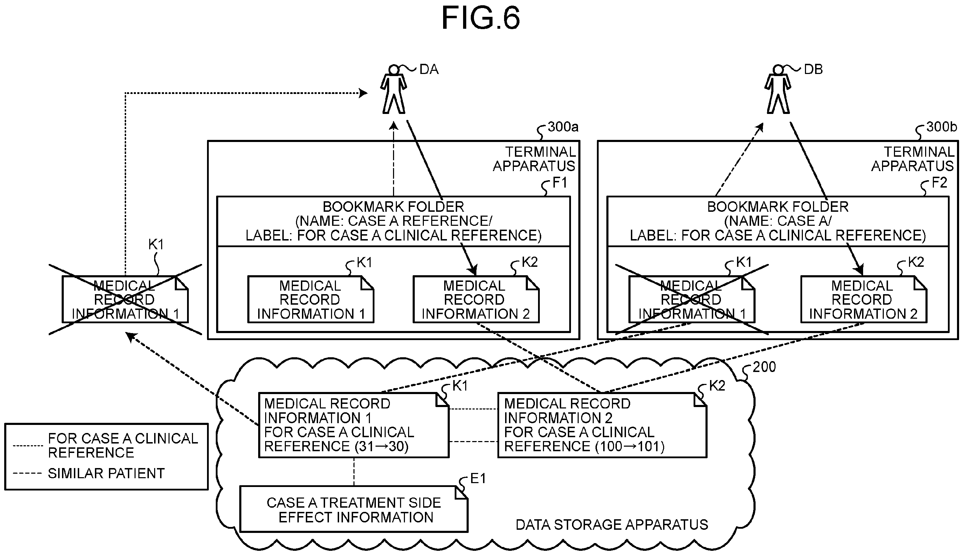

Next, a recommendation process for the data deletion is described with reference to FIG. 6. FIG. 6 is a diagram for describing one example of the recommendation process for the data deletion.

In FIG. 6, the users who use the bookmark function are the physician DA and the physician DB. In FIG. 6, "medical record information 2" K2 is added to "case A reference" F1 by the physician DB. In addition, "medical record information 2" K2 is stored in "case A" F2 by the physician DB. Furthermore, "medical record information 1" K1 is deleted from "case A" F2 by the physician DB.

Moreover, the label "for case A clinical reference" is assigned to "case A reference" F1 in the terminal apparatus 300a and "case A" F2 in the terminal apparatus 300b.

The score of "for case A clinical reference" in "medical record information 2" K2 of the data storage apparatus 200 is assumed to be "100". The score of "for case A clinical reference" in "medical record information 1" K1 of the data storage apparatus 200 is assumed to be "31".

In this case, the acquisition function 141 detects that "medical record information 2" K2 has been added to "case A reference" F1 in response to the operation of the physician DA. The acquisition function 141 then acquires information indicating that "medical record information 2" K2 has been added to "case A reference" F1. Next, the label assignment function 143 adds one to the score of "for case A clinical reference" in "medical record information 2" K2, and updates the score from "100" to "101".

The acquisition function 141 detects that "medical record information 1" K1 has been deleted with respect to "case A" F2 in response to the operation of the physician DB. The acquisition function 141 then acquires information indicating that "medical record information 1" K1 has been deleted. Next, the label assignment function 143 subtracts one from the score of "for case A clinical reference" in "medical record information 1" and updates the score from "31" to "30".

Next, the retrieval function 146 retrieves the relevant data of "medical record information 2" K2 pertaining to the operation of the physician DA. In FIG. 6, the retrieval function 146 retrieves the data of the similar patients who are similar to the patient pertaining to "medical record information 2" K2 as the relevant data. The retrieval function 146 retrieves the data assigned with the label "for case A clinical reference" assigned to "medical record information 2" K2 as relevant data by the operation of the physician DA.

Here, "medical record information 1" K1 is the data of the similar patient who is similar to the patient pertaining to "medical record information 2" K2. In addition, "medical record information 1" K1 is the data assigned with the label "for case A clinical reference" that is the same as the label of "medical record information 2" K2. In addition, "case A treatment side effect information" is the data of the similar patient who is similar to the patient pertaining to "medical record information 2" K2.

Therefore, the retrieval function 146 retrieves "medical record information 1" K1 and "case A treatment side effect information" E1 as the relevant data in "medical record information 2" K2. The retrieval function 146 associates "medical record information 2" K2 and "medical record information 1" K1 to the effect that they are the data of the similar patients. Similarly, the retrieval function 146 associates "medical record information 2" K2 and "case A treatment side effect information" E1 to the effect that they are the data of the similar patients.

Furthermore, since "medical record information 2" K2 and "medical record information 1" K1 are assigned with the same label "for case A clinical reference", the retrieval function 146 associates "medical record information 2" K2 and "medical record information 1" K1 to the effect that they are data assigned with the similar label. Although not illustrated, the retrieval function 146 performs the process similar to that for "medical record information 2" K2 pertaining to the operation information of the physician DA as for "medical record information 1" K1 pertaining to the operation information of the physician DB.

The presentation function 144 then presents the operation action for the data. In FIG. 6, the presentation function 144 checks the change in label score in a predetermined period regarding the data that is associated to the effect that the data is assigned with the label similar to that of "medical record information 2" K2.

For example, in FIG. 6, the score of "for case A clinical reference" in "medical record information 1" K1 decreases from "31" to "30". The presentation function 144 checks such a change in score in a predetermined period.

The presentation function 144 determines, for example, whether the decrease quantity in the score of "for case A clinical reference" in "medical record information 1" K1 in the predetermined period is over a predetermined threshold. If the decrease quantity in the score of "for case A clinical reference" in the predetermined period is over the threshold, the presentation function 144 specifies the users who manage the data using the bookmark folder assigned with the label "for case A clinical reference".

The presentation function 144 also generates a notification to encourage the user, among the specified users, who has "medical record information 1" K1 stored in the bookmark folder to delete "medical record information 1" K1 from that bookmark folder.

In FIG. 6, the physician DA manages the data using "case A reference" F1, which is the bookmark folder assigned with the label "for case A clinical reference". In addition, the physician DA has "medical record information 1" K1 stored in "case A reference" F1. Therefore, the presentation function 144 generates a notification to encourage the physician DA to delete "medical record information 2" K2 from "case A reference" F1.

Even if the physician DA deletes "medical record information 1" K1 from "case A reference" F1 in accordance with the recommendation of deletion, "medical record information 1" K1 will not be deleted from the data storage apparatus 200.

Here, the data with the decreasing label score is considered to be the data that is less important, such as old data. Thus, as described above, the presentation function 144 encourages the user to delete the data with the decreasing label score from the bookmark folder, thereby preventing the user from referring to outdated information in diagnosing a case.

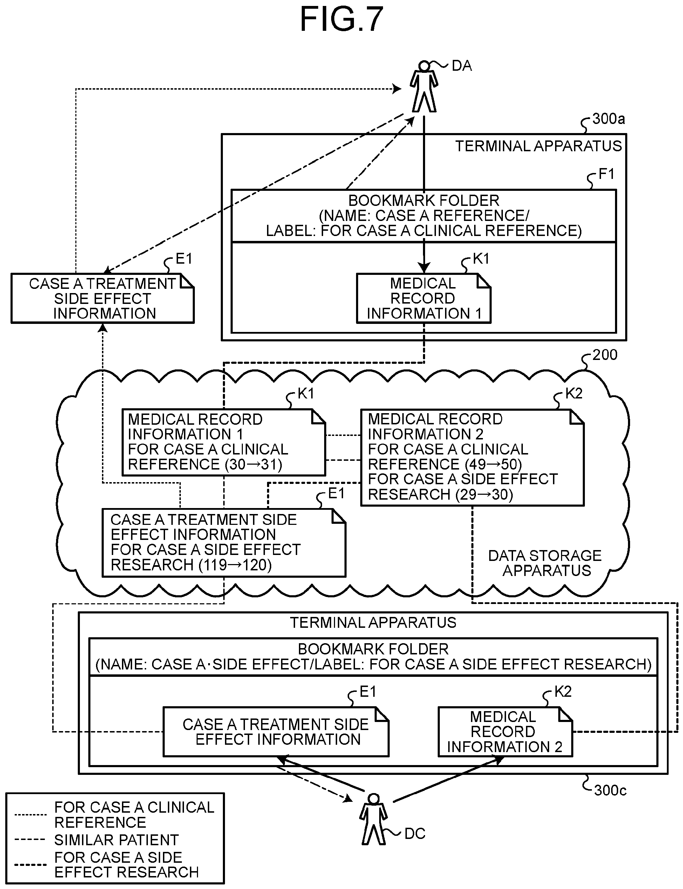

Next, with reference to FIG. 7, the recommendation process for the data addition of the data with the different label is described. FIG. 7 is a diagram for describing the recommendation process for the data addition of the data with the different label.

In FIG. 7, the label "for case A clinical reference" is assigned to "case A reference" F1. The label "for case A side effect research" is assigned to "case A/side effect" F3.

The score of "for case A clinical reference" in "medical record information 1" K1 of the data storage apparatus 200 is assumed to be "30". The score of "for case A clinical reference" in "medical record information 2" K2 of the data storage apparatus 200 is assumed to be "49". The score of "for case A side effect research" in "medical record information" K2 of the data storage apparatus 200 is assumed to be "29". The score of "for case A side effect research" in "case A treatment side effect information" E1 of the data storage apparatus 200 is assumed to be "119".

In FIG. 7, the users who use the bookmark function are the physicians DA and DC. In a manner similar to FIG. 4, "medical record information 1" K1 is added to "case A reference" F1 by the physician DA. In this case, the label assignment function 143 updates the score of "for case A clinical reference" in "medical record information 1" K1 from "30" to "31".

In addition, "case A treatment side effect information" E1 and "medical record information 2" K2 are added to "case A/side effect" F3 by the physician DC. In this case, the label assignment function 143 updates the score of "for case A side effect research" in "case A treatment side effect information" E1 from "119" to "120".

In addition, the label assignment function 143 updates the score of "for case A clinical reference" in "medical record information 2" K2 from "49" to "50". Moreover, the label assignment function 143 updates the score of "for case A side effect research" in "medical record information" K2 from "29" to "30".

In this case, the retrieval function 146 retrieves the relevant data of "medical record information 1" K1 pertaining to the operation information of the physician DA. In FIG. 7, the retrieval function 146 retrieves the data of the similar patients who are similar to the patient of "medical record information 1" K1 as the relevant data.

Here, "medical record information 2" K2 is the data of the similar patient who is similar to the patient pertaining to "medical record information 1" K1. In addition, "medical record information 2" K2 is the data assigned with the label "for case A clinical reference" that is the same as the label of "medical record information 1" K1. In addition, "case A treatment side effect information" is the data of the similar patient who is similar to the patient pertaining to "medical record information 1" K1.

Therefore, "medical record information 2" K2 and "case A treatment side effect information" E1 are retrieved as the relevant data by the retrieval function 146. The retrieval function 146 associates "medical record information 1" K1 and "medical record information 2" K2 to the effect that they are the data of the similar patients. Similarly, the retrieval function 146 associates "medical record information 1" K1 and "case A treatment side effect information" E1 to the effect that they are the data of the similar patients.

Furthermore, since "medical record information 1" K1 and "medical record information 2" K2 are assigned with the same label "for case A clinical reference", the retrieval function 146 associates "medical record information 1" K1 and "medical record information 2" K2 to the effect that they are data assigned with the similar label.

Since "medical record information 2" K2 and "case A treatment side effect information" E1 are assigned with the same label "for case A side effect research," the retrieval function 146 associates "medical record information 2" K2 and "case A treatment side effect information" E1 to the effect that they are the data assigned with the similar label.

Although not illustrated, the retrieval function 146 performs the process similar to that for "medical record information 1" K1 pertaining to the operation information of the physician DA as for "medical record information 2" K2 and "case A treatment side effect information" pertaining to the operation information of the physician DC.

The presentation function 144 then presents the action for the data. In FIG. 7, "medical record information 2" K2 is the hub data assigned with two labels, "for case A clinical reference" and "for case A side effect research". In this case, the presentation function 144 checks the change in the label score in a predetermined period regarding the data that is associated to the effect that the data is assigned with the label similar to that of "medical record information 2" K2, which is the hub data.

For example, in FIG. 7, the score of "for case A side effect research" in "case A treatment side effect information" E1 increases from "119" to "120". The presentation function 144 checks such a change in score in a predetermined period.

The presentation function 144 determines whether the increase quantity in the score of "for case A side effect research" in "case A treatment side effect information" E1 in a predetermined period is over a predetermined threshold. If the increase quantity in the score of "for case A side effect research" in the predetermined period is over the threshold, the presentation function 144 will then specify the users who manage the data using the bookmark folder with the same label as the label "for case A clinical reference" assigned to the bookmark folder pertaining to the operation information of the physician DA.

The presentation function 144 also generates a notification to encourage the user, among the specified users, who does not have "case A treatment side effect information" E1 to add "case A treatment side effect information" E1 to the bookmark folder.

In FIG. 7, the physician DA manages the data using "case A reference" F1, which is the bookmark folder assigned with the label "for case A clinical reference". In addition, the physician DA does not have "case A treatment side effect information" E1. For this reason, the presentation function 144 generates a notification to encourage the physician DA to add "case A treatment side effect information" E1 to "case A reference" F1.

The presentation function 144 may generate a notification to encourage the physician DA to create a folder of "for case A side effect research" and add "case A treatment side effect information" E1.

Thus, if the hub data exists, the presentation function 144 checks the label score about the data assigned with the same label as the hub data. The presentation function 144 then recommends the user to add if the score is increasing. This allows the presentation function 144 to encourage the user to also refer to the data assigned with the label different from that of the data on which the user performed the operation.

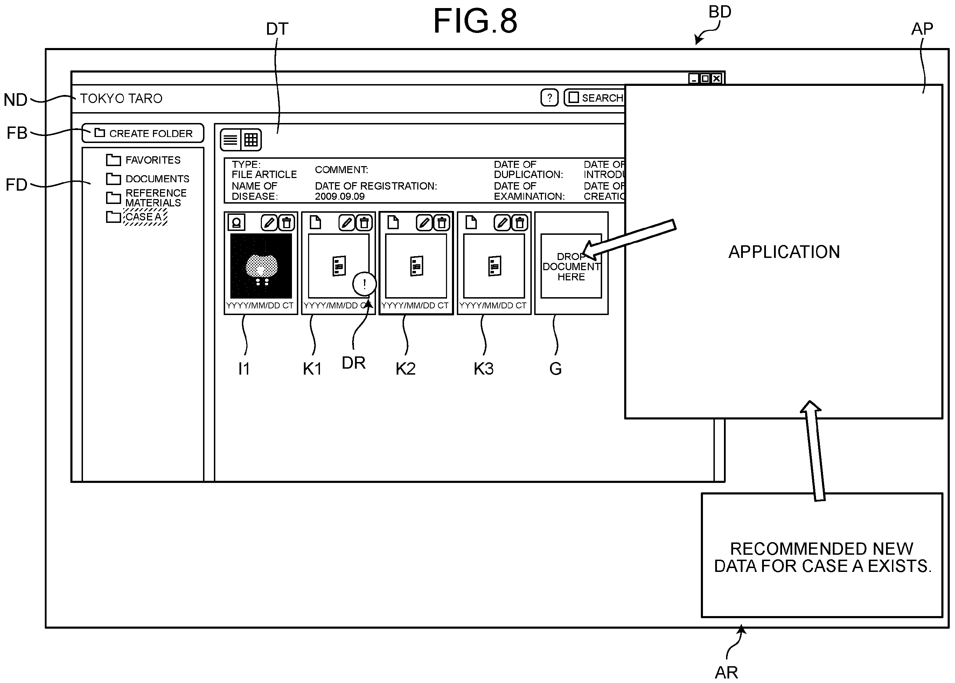

Next, the display control process for the terminal apparatus 300 is described with reference to FIG. 8. The output control function 147 of the medical information processing apparatus 100 controls the display process by the terminal apparatus 300. For example, the output control function 147 sends a display control instruction to the terminal apparatus 300 via the network NW to cause the display apparatus of the terminal apparatus 300 to display various kinds of information.

FIG. 8 is a diagram for describing one example of the display control process for the terminal apparatus 300. In FIG. 8, the output control function 147 sends the display control instruction to the terminal apparatus 300 to display the bookmark display screen BD.

The bookmark display screen BD in FIG. 8 includes the user name display column ND, the folder creation button FB, the folder display column FD, and a data display column DT. The user name display column ND, the folder creation button FB, and the folder display column FD are similar to those in FIG. 2 and therefore, the description is omitted.

The data display column DT is a display column that displays the data (files) stored in the bookmark folder selected by the user. FIG. 8 illustrates the data display column DT displaying the data stored in the bookmark folder "case A".

In the data display column DT in FIG. 8, medical image data I1 whose file name is "CT image data", "medical record information 1" K1, "medical record information 2" K2, "medical record information 3" K3, and data addition guide display G are displayed. In addition, a deletion recommendation DR is displayed for "medical record information 1" K1.

For example, if the presentation function 144 generates a notification to encourage the user to delete the data from the bookmark folder, the output control function 147 sends a display control instruction for the deletion recommendation DR to the terminal apparatus 300. This causes the deletion recommendation DR to be displayed overlapping with the data targeted by the deletion recommendation displayed in the data display column DT.

The bookmark display screen BD in FIG. 8 also displays an addition recommendation AR. For example, if the presentation function 144 generates a notification to encourage the user to add the data to the bookmark folder, the output control function 147 sends a display control instruction for the addition recommendation AR to the terminal apparatus 300. This causes the addition recommendation AR to be displayed on the bookmark display screen BD.

In FIG. 8, as the addition recommendation AR, it is displayed that there is data that is recommended to be added to the bookmark folder "case A".

In this case, the output control function 147 also sends out a display control instruction for the data addition guide display G. This causes the data addition guide display G to be displayed on the data display column DT. The data addition guide display G is a display to assist the process of adding the data to the bookmark folder.

For example, when the user clicks on the addition recommendation AR with the mouse or the like, an application AP for managing the data is activated. Next, an icon representing the data to be added is displayed on the display apparatus of the terminal apparatus 300 via the application AP. Then, for example, when the user drags and drops that icon onto the data addition guide display G with the mouse operation, the data to be added is added to the bookmark folder "case A".

Thus, when the output control function 147 causes the display apparatus of the terminal apparatus 300 to display the deletion recommendation DR and the addition recommendation AR, the user can easily delete old data and add new data, making it easier to manage the bookmark folder.

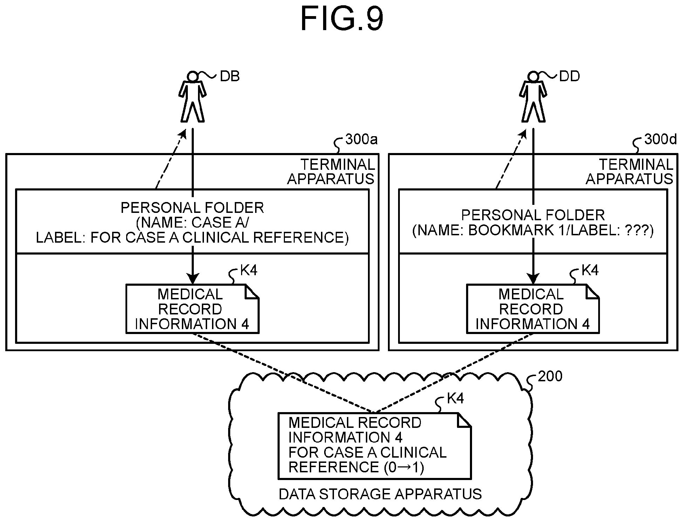

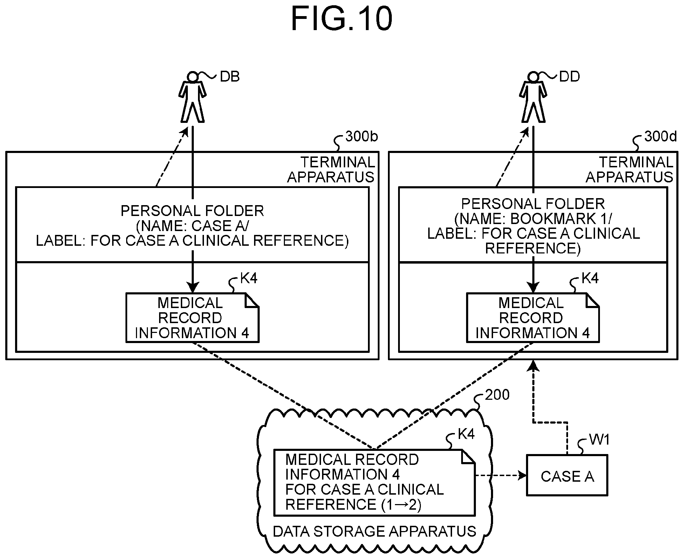

Next, with reference to FIG. 9 and FIG. 10, description is made of the label assignment process in a case where the label assignment by the analysis of the name of the bookmark folder is impossible. FIG. 9 and FIG. 10 are diagrams for describing one example of the label assignment process when the label assignment by the analysis of the name of the bookmark folder is impossible.

In FIG. 9 and FIG. 10, the users who use the bookmark function are the physician DB and a physician DD. The physician DD is a clinician and is a non-meticulous user. The terminal apparatus 300d is a terminal apparatus used by the physician DD.

Moreover, electronic medical record data K4 with the file name "medical record information 4" (hereinafter also referred to as " medical record information 4" K4) is added to "case A" F2 by the physician DB and "medical record information 4" K4 is added to a bookmark folder F4 with the folder name "bookmark 1" (hereinafter also referred to as "bookmark 1" F4) by the physician DD.

The label "for case A clinical reference" is assigned to "case A reference" F1. In addition, "bookmark 1" F4 is not assigned with the label. The score of "for case A clinical reference" in "medical record information 4" K4 is assumed to be "0".

In this case, the label assignment function 143 updates the score of "for case A clinical reference" in "medical record information 4" K4 from "0" to "1".

In this case, the analysis function 142 performs the analysis of "bookmark 1" F4, which is the name of the bookmark folder. Furthermore, the analysis function 142 performs a process for specifying the attributes of "bookmark 1" F4 and "medical record information 4" K4 with reference to the label setting information stored in the memory 130.

However, as illustrated in FIG. 9, "bookmark 1" is not the name representing the intended use of the data; therefore, even if the user understands what kind of data is stored in "bookmark 1", the analysis function 142 cannot specify the attributes of "bookmark 1" F4 and "medical record information 4" K4.

Therefore, in such a case, the analysis function 142 sends information to the extraction function 145 that the attribute could not be specified. The extraction function 145, to which the information that the attribute could not be specified was sent, extracts the keyword representing the characteristic of the data from the data stored in the bookmark folder whose attribute could not be specified.

In FIG. 10, the extraction function 145 performs a keyword extraction process on "medical record information 4" K4 using the known natural language processing technique or the like to extract the keyword. The extraction function 145 sends the extracted keyword "case A" W1 to the analysis function 142 as a summary.

Based on the label setting information and the summary sent out, the analysis function 142 specifies the attributes of the bookmark folder whose attributes could not be specified from the name of the bookmark folder and the data stored in that bookmark folder.

In FIG. 10, the analysis function 142 specifies the label "for case A clinical reference" as the attributes of "bookmark 1" F1 and "medical record information 4" K4 on the basis of the label setting information stored in the memory 130 and the summary "case A" W1 sent out by the extraction function 145.

Next, the label assignment function 143 assigns the label "for case A clinical reference" representing the attribute of "medical record information 4" K4. In addition, the label assignment function 143 updates the score of "for case A clinical reference" in "medical record information 4" K4 from "1" to "2". The label assignment function 143 assigns the label "for case A clinical reference" representing the attribute of "bookmark 1" F4 to "bookmark 1" F4 .

Thus, even in the case where, for example, the non-meticulous user uses the name that does not suit the intended purpose of the data stored in the bookmark folder as the name of the bookmark folder, the label assignment function 143 can assign the label to the bookmark folder and the data stored in the bookmark folder.

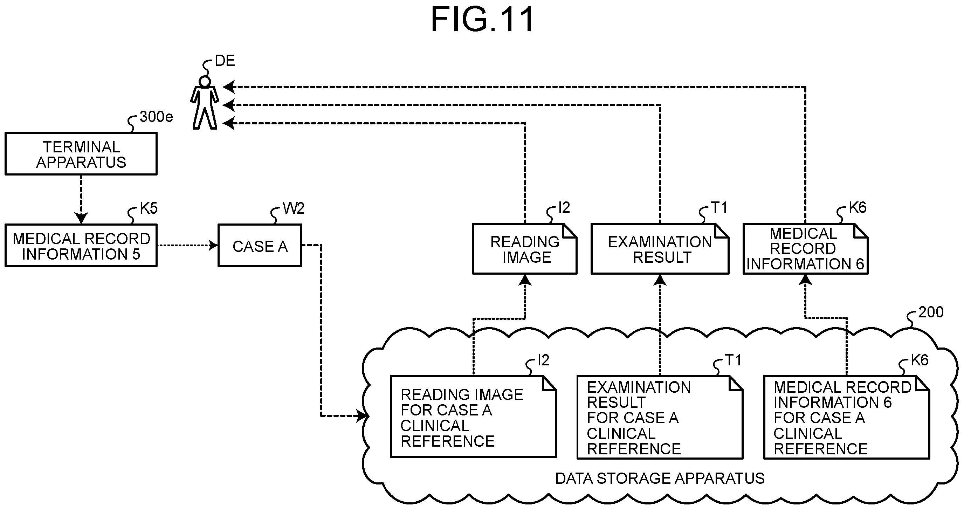

Next, the recommendation process for the users who do not use the bookmark function is described with reference to FIG. 11. FIG. 11 is a diagram for describing one example of the recommendation process for the users who do not use the bookmark function.

The medical information processing system 1 in FIG. 11 includes the data storage apparatus 200 and the terminal apparatus 300e. In FIG. 11, the user of the medical information processing system 1 is a physician DE. The physician DE is a user who does not use the bookmark function. One of the possible users who do not use the bookmark function is a medical practitioner. The terminal apparatus 300e is a terminal apparatus used by the physician DE. Described below is a case in which the physician DE creates electronic medical record data K5 with the file name "medical record information 5" (hereinafter also referred to as "medical record information 5" K5) using the terminal apparatus 300e.

In this case, the acquisition function 141 acquires information about the operation of the user who does not use the bookmark function. In FIG. 11, the acquisition function 141 acquires information representing that "medical record information 5" K5 has been created on the basis of the operation of the terminal apparatus 300e by the physician DE.

In FIG. 11, the acquisition function 141 acquires the information that the data has been created, but the information to be acquired is not limited to this. For example, the acquisition function 141 may acquire the information that the data has been browsed.

Next, the extraction function 145 extracts words related to a predetermined word from the data pertaining to the user operation. In FIG. 11, the extraction function 145 performs the keyword extraction process on the "medical record information 5" K5 created using the known natural language processing technique or the like, thereby extracting the keyword. The extraction function 145 sends the extracted keyword "case A" W2 to the analysis function 142 as a summary.

The analysis function 142 specifies the label related to the data pertaining to the user operation on the basis of the label setting information and the summary sent out. In FIG. 11, the analysis function 142 specifies the label "for case A clinical reference" as the label related to "medical record information 5" K5, based on the label setting information stored in the memory 130 and the summary "case A" W2 sent from the extraction function 145.

Next, the retrieval function 146 retrieves the data assigned with the specified label. In FIG. 11, the retrieval function 146 retrieves the data to which the label "for case A clinical reference" is assigned, from the data in the data storage apparatus 200.

The presentation function 144 then generates a notification to encourage the user to refer to the data retrieved by the retrieval function 146 that meets the condition (for example, the label score exceeds the threshold). In FIG. 11, the presentation function 144 generates a notification to encourage the physician DE to refer to medical image data I2 whose file name is "reading image", examination result data T1 whose file name is "examination result", and electronic medical record data K6 whose file name is "medical record information 6".

This allows the presentation function 144 to encourage the user who does not use the bookmark function to refer to the data that is estimated to be highly important.

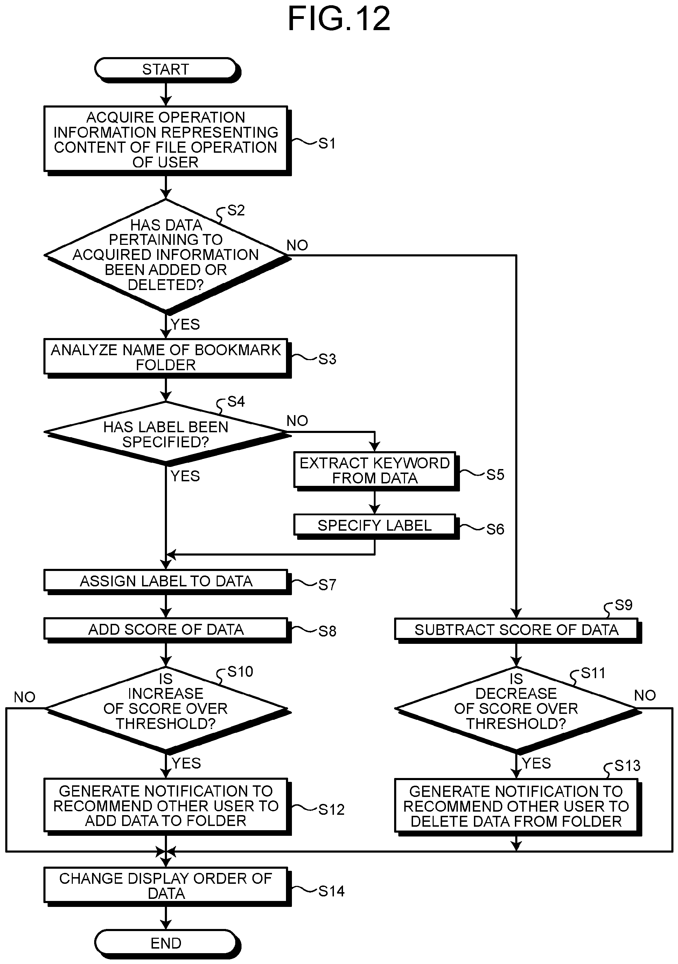

Next, the processes to be performed by the medical information processing apparatus 100 according to this embodiment will be described. First, the process for the users who use the bookmark function is described with reference to FIG. 12. FIG. 12 is a flowchart expressing one example of the process for the user who uses the bookmark function to be performed by the medical information processing apparatus 100 according to the embodiment.

First, the acquisition function 141 acquires operation information representing the contents of the file operation of the user (step S1). For example, the acquisition function 141 monitors the bookmark folders created by the users.

Next, the acquisition function 141 detects that the data has been added to or deleted from the bookmark folder on the basis of the user's operation of the terminal apparatus 300. Then, the acquisition function 141 acquires information representing that the data has been added to or deleted from the bookmark folder on the basis of the user's operation of the terminal apparatus 300.

Next, the analysis function 142 detects whether the data has been added to or deleted from the bookmark folder pertaining to the operation information acquired at step S1 (step S2).

If the data has been added (Yes at step S2), the analysis function 142 analyzes the name of the bookmark folder to which the data has been added (step S3). For example, the analysis function 142 analyzes the names of the bookmark folder using the known natural language processing technique.

Next, the analysis function 142 determines whether the attribute of the data has been specified from the analysis result (step S4). For example, the analysis function 142 determines whether the name of the label representing the attribute corresponding to the analysis result exists, with reference to the label setting information stored in the memory 130.

If the attribute has not been specified (No at step S4), the extraction function 145 performs the keyword extraction process from the data added to the bookmark folder (step S5). For example, the extraction function 145 extracts a keyword from the data using the known natural language processing technique. The extraction function 145 then sends the extraction result to the analysis function 142 as a summary.

Next, the analysis function 142 specifies the attributes of the bookmark folder and the data added to that bookmark folder, based on the label setting information and the summary sent out (step S6). Subsequently, the label assignment function 143 assigns the label representing the attribute specified at step S6 to the bookmark folder and the data added to that bookmark folder (step S7).

Next, the label assignment function 143 performs a process of adding the score of the assigned label regarding the data added to the bookmark folder (step S8). Subsequently, the presentation function 144 determines whether the increase quantity in the label score in a predetermined period is over a predetermined threshold regarding the data added to the bookmark folder (step S10).

If the increase quantity in the score is not over the threshold (No at step S10), the process advances to step S14. On the other hand, if the increase quantity in the score is over the threshold (Yes at step S10), the presentation function 144 specifies the users with the bookmark folder assigned with the label similar to that assigned at step S7. The presentation function 144 also generates a notification to recommend the user, among the specified users, who does not have the data whose increase quantity in the score is over the threshold to add the data whose increase in score is over the threshold (step S12).

Next, the output control function 147 sends to the terminal apparatus 300 used by the user pertaining to the information acquired at step S1, a display control instruction to change the display order of the data in the bookmark folder on the basis of the label score (step S14), and ends this process.

If the data has been deleted at step S2 (No at step S2), the label assignment function 143 specifies the label assigned to the bookmark folder pertaining to the information acquired at step S1. Next, the label assignment function 143 performs a process of subtracting the score of the specified label regarding the data deleted from the bookmark folder (step S9).

Subsequently, the presentation function 144 determines whether the decrease quantity in the label score in a predetermined period is over a predetermined threshold regarding the data deleted from the bookmark folder (step S11) .

If the decrease quantity in the score is not over the threshold (No at step S11), the process advances to step S14. On the other hand, if the decrease quantity in the score is over the threshold (Yes at step S11), the presentation function 144 specifies the users with the bookmark folder assigned with the label similar to that assigned at step S7.

The presentation function 144 also generates a notification to recommend the user, among the specified users, who has the data whose decrease quantity in the score is over the threshold stored in the bookmark folder to delete the data (step S13) and then the process advances to step S14.

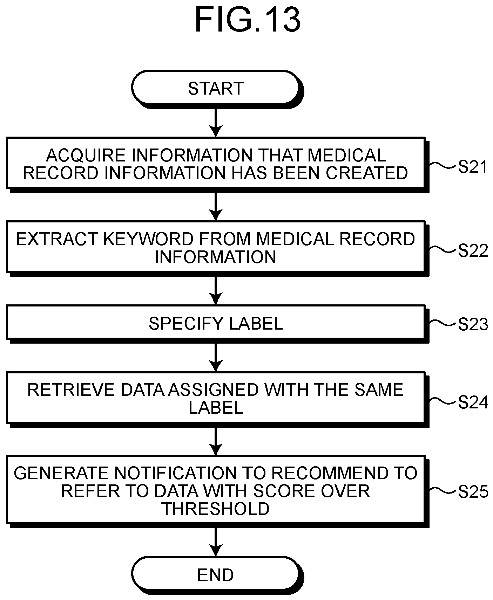

Next, the process for the users who do not use the bookmark function is described with reference to FIG. 13. FIG. 13 is a flowchart expressing one example of the process for the user who does not use the bookmark function to be performed by the medical information processing apparatus 100 according to the embodiment.

First, the acquisition function 141 acquires information that medical record information has been created by the user who does not use the bookmark function (step S21). For example, the acquisition function 141 acquires the information that medical record information has been created, based on the user's operation of the terminal apparatus 300.

Next, the extraction function 145 extracts a keyword from the medical record information pertaining to the information acquired at step S1 (step S22). For example, the extraction function 145 extracts a keyword from the data using the known natural language processing technique. The extraction function 145 then sends the extraction result to the analysis function 142 as a summary.

Next, the analysis function 142 specifies the attribute of the created medical record information, based on the label setting information and the sent summary (step S23). Subsequently, the retrieval function 146 retrieves the data assigned with the same label as the label representing the attribute specified at step S23 from among the data stored in the data storage apparatus 200 (step S24).

Next, the presentation function 144 generates a notification to encourage the user, who does not use the bookmark function, to refer to the data whose label score is over the threshold among the data retrieved at step S24 (step S25), and ends this process.

In FIG. 13, the case in which the user creates the medical record information is described as an example; however, the data to be created by the user is not limited to the medical record information. In another example, the data to be created by the user may be a reading report or the like. In FIG. 13, the case in which the user creates the data is described; however, the user's operation on the data is not limited to creation. In another example, the user's operation on the data may be browsing.

As described above, the medical information processing apparatus 100 according to this embodiment assigns the label representing the attribute to the bookmark folder on the basis of the file the user intends to browse or operate and the bookmark folder storing such a file, which are managed by each user, and based on the file stored in the bookmark folder with the label with the same attribute, sets the condition pertaining to the presentation of the file.

Here, for example, if there is a file that is frequently browsed by multiple users, it can be presumed that the file is of high importance. Therefore, the medical information processing apparatus 100 according to this embodiment can, for example, encourage the target user to refer to the file that is estimated to be of high importance as described above by making the file to which more labels than a certain number are assigned in a predetermined period the target of the presentation of the action of adding the file. In another example, if multiple users delete the file from their terminal apparatuses 300, it can be presumed that the file is medical information whose importance has decreased. Therefore, the medical information processing apparatus 100 according to this embodiment can, for example, encourage the target user to delete the medical information that is estimated to become less important as described above by making the file to which fewer labels than a certain number are assigned in a predetermined period the target of the presentation of the action of deleting the file. Thus, the users are less likely to overlook the existence of new information or continue to keep having outdated information. In other words, the medical information processing apparatus 100 according to this embodiment can assist the proper management of files pertaining to the medical information.

Based on the name of the folder as the target of the file operation included in the operation information, the medical information processing apparatus 100 according to this embodiment specifies the attribute that represents the characteristic of the file corresponding to the file stored in that folder. The medical information processing apparatus 100 according to this embodiment also assigns the label representing the specified attribute to the folder and the file stored in the folder, and presents the action for the file operation on the basis of the number of labels with the same attribute assigned to the medical information.

For example, in the case where the score representing the number of labels with the same attribute of the file has increased over a threshold in a predetermined period, the medical information processing apparatus 100 according to this embodiment presents the action of adding the file to the folder to the target user who manages the file using the folder assigned with the label with the same attribute as the label of the medical information.

Here, the increase in the score of the label assigned to a particular file means that many users have added that file to their bookmark folders. In other words, the increase in the label score indicates that file is becoming more important. In this way, by encouraging the target user to add the data that is estimated to become more important, the medical information processing apparatus 100 according to this embodiment can prevent the user from overlooking the important file.

For example, in the case where the score representing the number of labels assigned to the file has decreased over a threshold in a predetermined period, the medical information processing apparatus 100 according to this embodiment presents the action of deleting the file from the folder to a particular user who manages the file using the folder assigned with the label with the same attribute as the label of the medical information.

Here, the decrease in the score of the label assigned to a particular file means that many users have deleted that file from their bookmark folders. In other words, the decrease in the label score indicates that file is becoming less important (information has become outdated). Thus, by encouraging the user to delete the data that is estimated to become less important, the medical information processing apparatus 100 according to this embodiment can prevent a situation in which the user makes a diagnosis based on old information.

If the attribute of the folder cannot be specified from the name of the folder, the medical information processing apparatus 100 according to this embodiment extracts a keyword from the file that indicates the characteristic of that file. The medical information processing apparatus 100 according to this embodiment also specifies the attribute of the folder on the basis of the extracted keyword.

This allows the medical information processing apparatus 100 according to this embodiment to assign the label to the folder and the data stored in that folder, even if the attribute of the folder could not be specified from the name of the folder.

In addition, the medical information processing apparatus 100 according to this embodiment retrieves the relevant medical information associated with the files of the files stored in the folder, and if there is a relevant file whose number of labels has increased over a threshold in a predetermined period, the medical information processing apparatus 100 presents the action of adding the relevant file to the folder to the target user who manages the file using the folder assigned with the label with the same attribute as the label of the file.

Thus, for example, the file assigned with the label different from the label assigned to the bookmark where the file pertaining to the operation information of the target user is stored can be recommended to the target user. Therefore, the medical information processing apparatus 100 according to this embodiment, for example, can recommend the information for research to users who have few opportunities to see the information for research.

The above-described embodiments can also be implemented by modifying some of the structures or functions that each apparatus has, as appropriate. In view of this, some modifications of the above-mentioned embodiments are described below as other embodiments. Note that described below are the points that differ from the above-mentioned embodiments, and the detailed description of the points in common with the aforementioned ones is omitted. The modifications below may be implemented individually or in combination as appropriate.

### First modification

In the description of the above embodiments, the user gives a desired name to the bookmark folder. However, the name selected by the user from the list may be used as the name of the bookmark folder.

In this modification, for example, when a user presses the folder creation button FB in FIG. 2 or FIG. 8, the output control function 147 of the medical information processing apparatus 100 sends to the terminal apparatus 300 used by the user, a display control instruction to display a list of names of the bookmark folder. The user selects one name of the bookmark folder from the list through the terminal apparatus 300.

This allows the user to use the selected name of the bookmark folder as the name of his or her bookmark folder. Here, in this modification, the names of the bookmark folder to be registered in the list are associated with the labels registered in the label setting information in the memory 130.

Therefore, in this modification, when the user has selected one name of the bookmark folder from the list, the label assignment function 143 assigns the label corresponding to the selected name of the bookmark folder to the created bookmark folder.

By the medical information processing apparatus 100 according to this modification, the processing burden on the processing circuitry 140 can be reduced because the process of analyzing the name of the bookmark folder is no longer necessary.

### Second modification

In the description of the above embodiments, the presentation function 144 generates a notification to encourage the user to add the data with the increasing label score to the bookmark folder to which that label is assigned. However, the presentation function 144 may alternatively generate a notification to encourage the user to add more recent data to the bookmark folder.

In this modification, for example, the presentation function 144 compares the data stored in the bookmark folder with the data with the same name stored in the data storage apparatus 200. The presentation function 144 then checks the date and time at which the data was updated. If the data with the same name stored in the data storage apparatus 200 is more recent, the presentation function 144 generates a notification to encourage the user to replace the data stored in the bookmark folder with the data with the same name stored in the data storage apparatus 200.

The medical information processing apparatus 100 according to this modification can prevent the situation in which the user overlooks the fact that data has been updated.

### Third modification

In the embodiments described above, the analysis function 142 analyzes the name of the bookmark folder. However, the analysis function 142 may analyze the structure of the bookmark folder. One example of the structure of the bookmark folder is the structure of a subfolder/data group that makes up the bookmark folder.

Here, the bookmark folders created by a clinician are considered to be divided into folders by case, for example, "for case A reference," "for case B reference," and so on. The number of pieces of data stored in the bookmark folder is estimated to be 100 or less.

On the other hand, the bookmark folders created by a research physician are considered to be divided into detailed folders, for example, "case A - new drug A clinical trial", "case A - side effects", etc. The number of pieces of data stored in the bookmark folder is estimated to exceed 100.

Therefore, by analyzing the structure in addition to the name of the bookmark folder, the analysis function 142 can estimate the attributes of the users (for example, whether the user is a clinician or a researcher), etc. In this modification, the analysis function 142 also takes into account the attributes of the users to specify the labels to be assigned to the bookmark folders and data.

According to this modification, the bookmark folders can be assigned with more suitable labels.

### Fourth modification

In the embodiments described above, the medical information processing apparatus 100 has the acquisition function 141, the analysis function 142, the label assignment function 143, the presentation function 144, the extraction function 145, the retrieval function 146, and the output control function 147. However, the data storage apparatus 200 may alternatively have some or all of these functions.

According to this modification, the medical information processing system 1 can be constructed to suit the environment by centralizing or distributing the processes.

Each component of each apparatus illustrated in the above embodiments is conceptual in terms of function, and does not necessarily have to be physically configured as illustrated in the drawings. In other words, the specific form of dispersion and integration of the apparatuses is not limited to that illustrated in the figure, but can be configured by functionally or physically dispersing or integrating all or some of them in arbitrary units according to various loads and usage conditions. Furthermore, all or any part of each processing function performed by each apparatus can be realized by a CPU and a computer program analyzed and executed by the CPU, or by hardware using wired logic.

The processing methods described in the above embodiments can also be realized by executing a computer program prepared in advance on a computer such as a personal computer or workstation. This computer program can be distributed via the Internet or other networks. This computer program can also be recorded on a computer-readable recording medium, such as a hard disk, flexible disk (FD), CD-ROM, MO, or DVD, and executed by being read out from the recording medium by a computer.

According to at least one of the embodiments described above, the proper management of the medical information can be assisted.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the spirit of the inventions. The accompanying claims and their equivalents are intended to cover such forms or modifications as would fall within the scope and spirit of the inventions.

With respect to the above embodiments and others, the following notes are disclosed as an aspect and selective features of the invention.

## Claims

1. A medical information processing apparatus (100) comprising:
an assignment unit (143) configured to, based on files pertaining to medical information that users intend to browse or operate, and folders that store the files therein, the files and the folders being managed by the respective users, assign a label representing an attribute to each of the folders; and
a setting unit (144) configured to, based on the files stored in the folders with the labels with the same attribute, set a condition pertaining to presentation of the file.

2. The medical information processing apparatus (100) according to claim 1, wherein the setting unit (144) is configured to, based on a first file corresponding to the file that a part of first users among the users stores in the folder, set a condition to present an action of adding the first file to the folder to second users who do not have the first file stored in the folder.

3. The medical information processing apparatus (100) according to claim 2, further comprising a presentation unit (144) configured to present an action of a file operation pertaining to the presentation of the file to the second user, wherein
the presentation unit (144) is configured to present the action of adding the first file to the folder to the second user, based on the set condition.

4. The medical information processing apparatus (100) according to claim 3, further comprising an acquisition unit (141) configured to acquire operation information representing a content of an operation of the user for the file, wherein
the presentation unit (144) is configured to present the action to the second user, based on the operation information of the first user in the operation information acquired about the users and the set condition that is set.

5. The medical information processing apparatus (100) according to claim 4, wherein the acquisition unit (141) is configured to acquire the operation information expressing the content of the file operation for the file or the folder that stores a link capable of accessing the file.

6. The medical information processing apparatus (100) according to claim 5, further comprising:
a specification unit (142) configured to specify the attribute representing a characteristic of the folder, based on a name of the folder as a target of the file operation included in the operation information; and
a counting unit (144) configured to count the number of labels, for each attribute, assigned to the files or the folders storing the files, for each file, wherein
the assignment unit (143) is configured to assign the label representing the attribute specified by the specification unit (142) to each of the folders and the files stored in the folders,
the counting unit (144) is configured to count the number of labels with the same attribute assigned to the files, and
based on the counted number of labels with the same attribute assigned to the files and the condition that is set, the presentation unit (144) is configured to present the action of the file operation for the folder to the second user.

7. The medical information processing apparatus (100) according to claim 6, wherein
the setting unit (144) is configured to set, as the condition, that an increase quantity of the number of labels with the same attribute assigned to the first file in a predetermined period is over a threshold, and
when the condition is satisfied, the presentation unit (144) is configured to present the action of adding the first file to the folder to the second user who manages the file different from the first file using the folder assigned with the label with the same attribute as the label set in the condition.

8. The medical information processing apparatus (100) according to claim 6, wherein
the setting unit (144) is configured to set, as the condition, that a decrease quantity of the number of labels with the same attribute assigned to a second file corresponding to the file that a part of the second users stores in the folder, in a predetermined period is over a threshold, and
when the condition is satisfied, the presentation unit (144) is configured to present an action of deleting the second file from the folder to the second user who manages the second file using the folder assigned with the label with the same attribute as the label set in the condition.

9. The medical information processing apparatus (100) according to claim 6, further comprising an extraction unit (145) configured to extract a keyword representing a characteristic of the file from the file, wherein
the specification unit (142) is configured to specify the attribute, based on the keyword extracted by the extraction unit (145).

10. The medical information processing apparatus (100) according to claim 6, further comprising a retrieval unit (146) configured to retrieve a relevant file related to a third file that the second user stores in the folder, wherein
when, as the condition, the relevant file in which the number of labels with the same attribute increases over the threshold in the predetermined period exists, the presentation unit (144) is configured to present an action of adding the relevant file to the folder to the second user who does not have the relevant file stored in the folder.

11. The medical information processing apparatus (100) according to claim 4, wherein the presentation unit (144) is configured to derive the action of the file operation, based on the operation information of the first user, in the operation information acquired about the users, assuming that the user who performs the file operation for the file more frequently than or as frequently as a threshold is the first user.

12. A medical information processing system (1) comprising a memory apparatus (200) configured to store medical information therein, one or a plurality of terminal apparatuses (300) configured to, by using a folder created for each user and being capable of storing the medical information or a link to the medical information as a file, receive a file operation of the user for the file, and a medical information processing apparatus (100), wherein the medical information processing apparatus (100) includes:
an assignment unit (143) configured to, based on files pertaining to medical information that users intend to browse or operate, and folders that store the files therein, the files and the folders being managed by the respective users, assign a label representing an attribute to each of the folders; and
a setting unit (144) configured to, based on the files stored in the folders with the labels with the same attribute, set a condition pertaining to presentation of the file.
